# EUROPEAN PATENT SPECIFICATION

(11) **EP 1 527 758 B1**
(45) Date of publication and mention of the grant of the patent: **17.01.2007**
(21) Application number: 03425710.5
(22) Date of filing: 31.10.2003
(51) Int. Cl.: A61F 2/42

(54) **Articular prosthesis for metacarpus-phalangeal or interphalangeal use**
Gelenksprothese für metakarpale oder interphalangäre Gelenke
Prothèse articulaire pour articulations metacarpophalangiennes ou interphalangiennes

(43) Date of publication of application: 04.05.2005
(73) Proprietor: ORTHOFIX S.r.l., 37012 Bussolengo VR (IT)
(72) Inventor: Venturini, Daniele, 37064 Povegliano Veronese (Verona) (IT); Corradi, Maurizio, 43100 Parma (IT); Rossi, Luigi, 37019 Peschiera del Garda (IT)
(74) Representative: Botti, Mario

(56) References cited:
- FR-A- 2 237 613
- US-A- 3 805 302

## Description

### Field of application

The present invention relates, in its more general aspect, to an articular prosthesis for metacarpus-phalangeal or interphalangeal use, which is thus used in arthroplasty, i.e. in the reconstruction of bone structures and of the different articular components, aiming at moving again the joint, after reconstructing the respective muscles, tendons, ligaments and the other limp tissues. This prosthesis is implanted after traumatic events or joint degenerative diseases such as the rheumatoid arthritis.

In particular, this invention relates to an articular prosthesis reconstructing a joint between the metacarpus and a phalanx or between two phalanxes, i.e. substantially comprising a first portion or shank to be associated to the proximal bone and a second portion or shank to be associated to the distal bone, these two shanks being connected to a half part of an articulated joint respectively, said two half parts being hinged to each other.

### Prior art

Several articular prosthesises are known in the hand and foot arthroplasty field.

Initially, the shanks had nail-or-screw-shaped ends to be inserted in the corresponding distal and proximal bones. Afterwards, to ensure a longer life and some reliability, the shank-bone union has been realised through pressure connection means in so-called "fasteners" or inserts, initially inserted in the metacarpal or phalangeal canal to be osteointegrated therein.

Nevertheless, given the limited area available in the bone canal to insert these fasteners, they have essentially a simple conical shape and they are not suitable for a possible prosthesis replacement which might be required by the articulated joint wear. In practise, in order to replace the joint, it is generally necessary to extract the existing fasteners and to insert therein other fasteners, generally greater in size, but this is not always possible.

In addition, for example, US 3, 805, 302 discloses a metacarpus-phalangeal or interphalangeal articular prosthesis on which the preamble of claim 1 is based.

### Summary of the invention

The problem underlying the present invention is to provide an articular prosthesis for metacarpus-phalangeal or interphalangeal use, capable of overcoming the drawbacks mentioned with reference to the prior art.

The problem is solved, according to the present invention, by an articular prosthesis, fur metacarpus-phalangeal or interphalangeal use, according to claim 1.

Further features and the advantages of the articular prosthesis, for metacarpus-phalangeal or interphalangeal use, according to the present invention, will be more apparent from the following description of an embodiment thereof made with reference to the attached drawings, given by way of indicative and non-limiting example.

### Brief description of the drawings

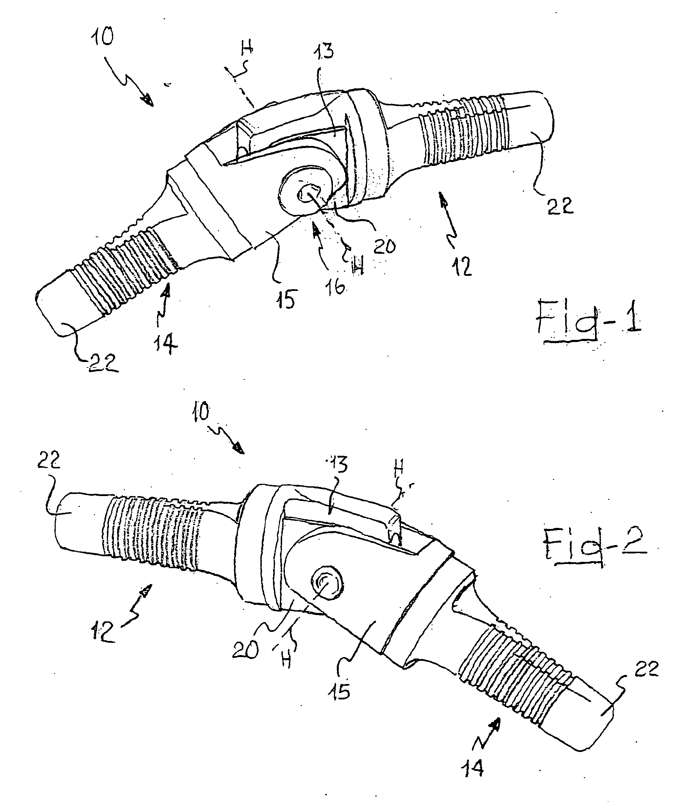

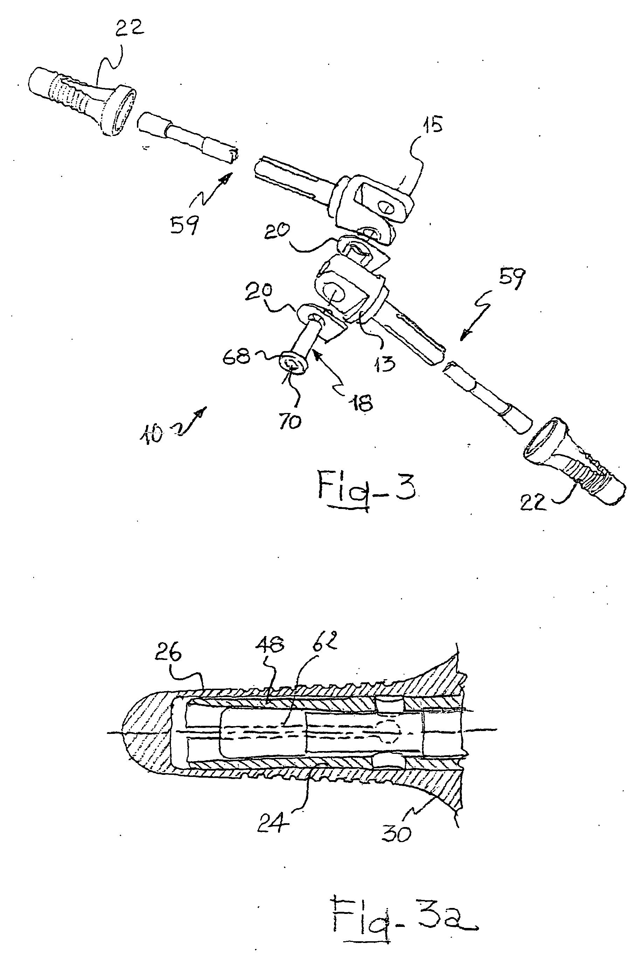

Figure 1 is a schematic perspective view of an articular prosthesis for metacarpus-phalangeal or interphalangeal use according to the invention.
Figure 2 is a schematic perspective view of the articular prosthesis of figure 1, from a different lookout point.
Figure 3 is an exploded perspective view of the prosthesis of figure 1.
Figure 3a shows an enlarged-scale cross section of a part of the prosthesis of figure 1.

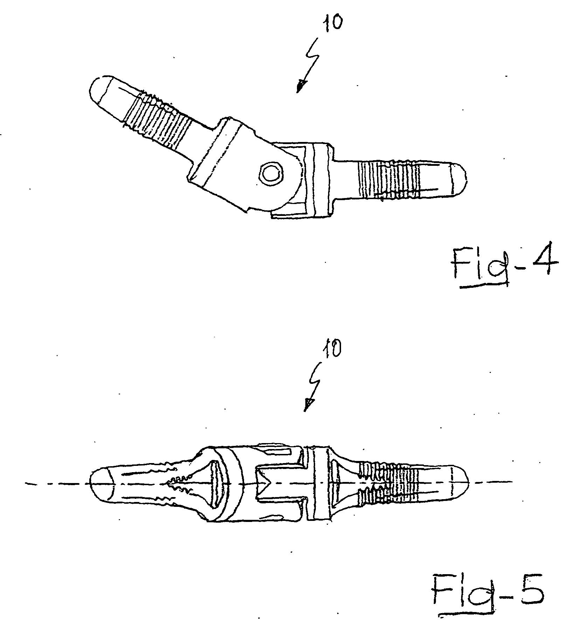

Figure 4 is a side-elevation view of the prosthesis of figure 1.
Figure 5 is a plan view from above of the prosthesis of figure 4.

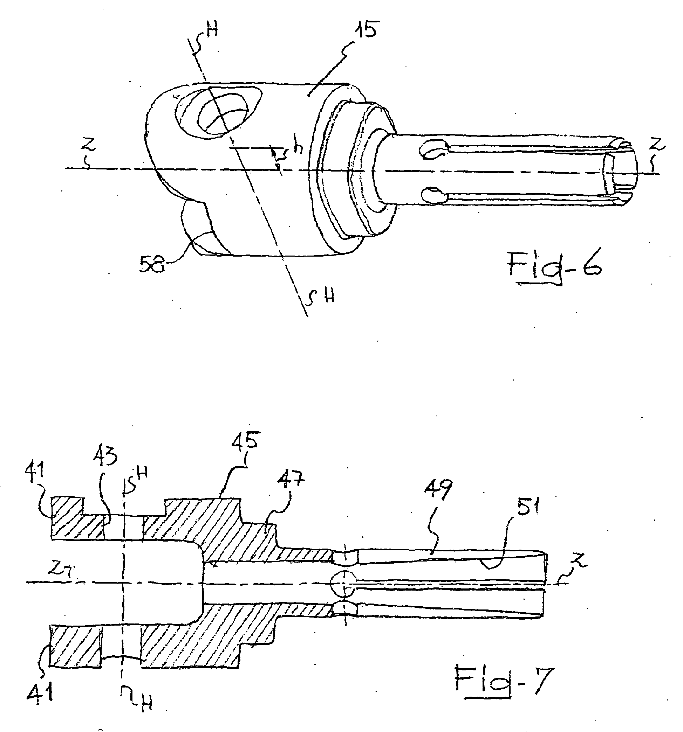

Figure 6 is an enlarged-scale perspective view of a detail of the prosthesis of figure 1.
Figure 7 shows a cross section of the detail of figure 6.

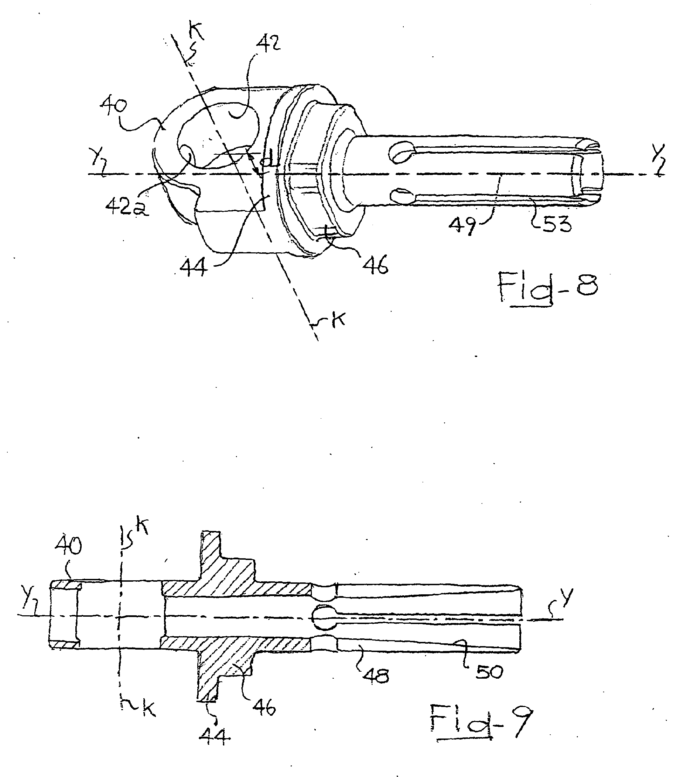

Figure 8 is an enlarged-scale perspective view of another detail of the prosthesis of figure 1.
Figure 9 shows a cross section of the detail of figure 8.
Figure 10 is a perspective view of a further detail of the prosthesis of figure 1.
Figure 10a is an enlarged-scale perspective view of a detail of figure 10.
Figure 10b is a plan view of the detail of figure 10a.
Figure 10c is a side-elevation view of the detail of figure 10a.
Figure 10d is a side-elevation view of the detail of figure 10a, taken from an opposite lookout point with respect to figure 10c.
Figure 10e is a cross section of the detail of figure 10b, taken according to the traced B-B plane of figure 10b.

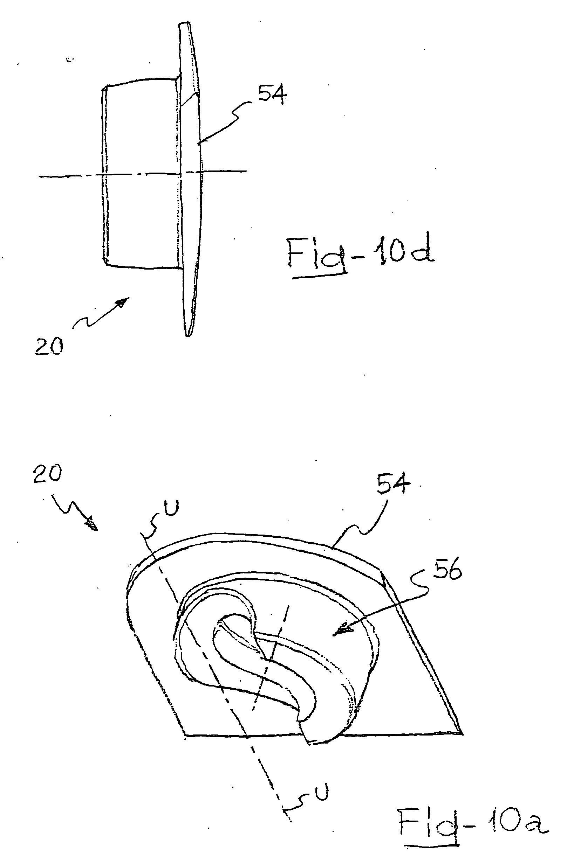

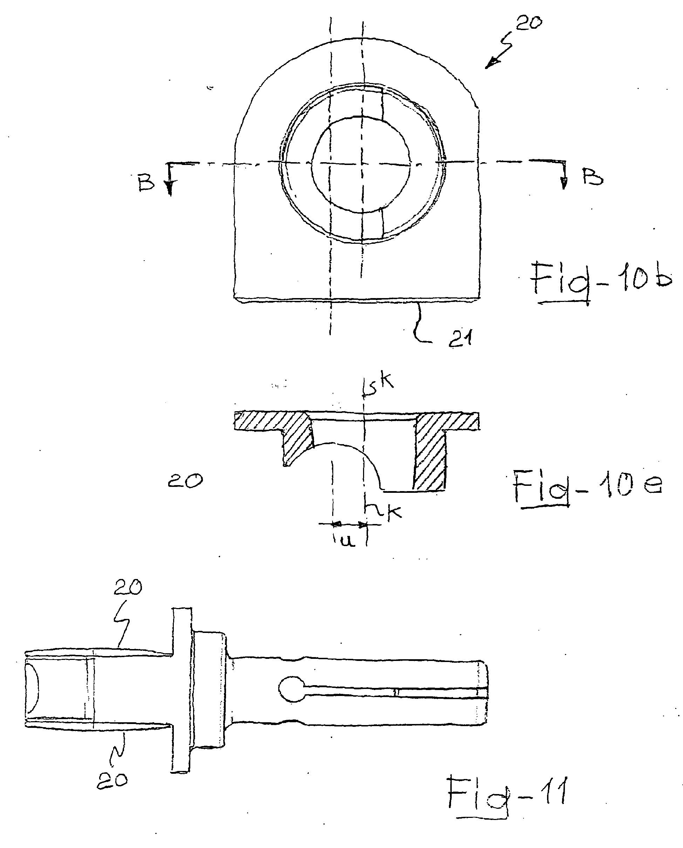

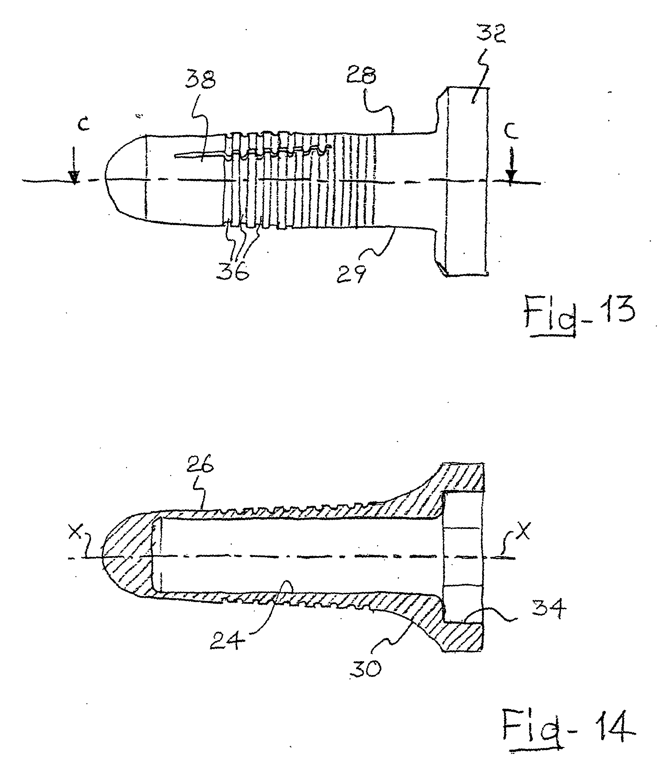

Figure 11 is a side-elevation view of the detail of figure 10.

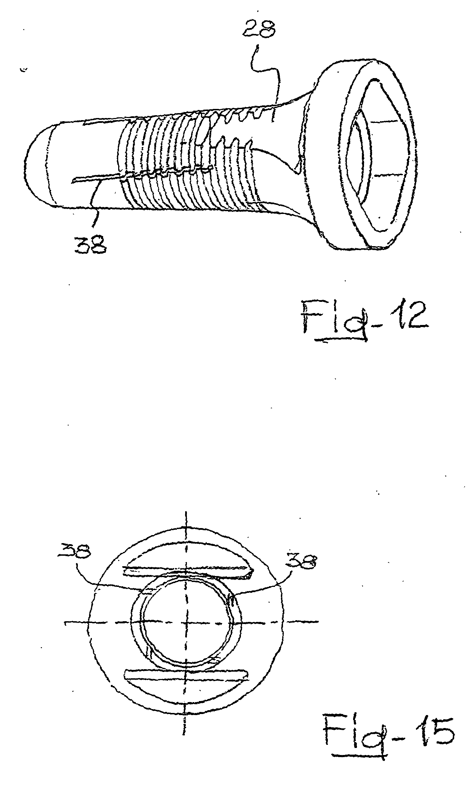

Figure 12 is an enlarged-scale perspective view of a further detail of the prosthesis of figure 1.

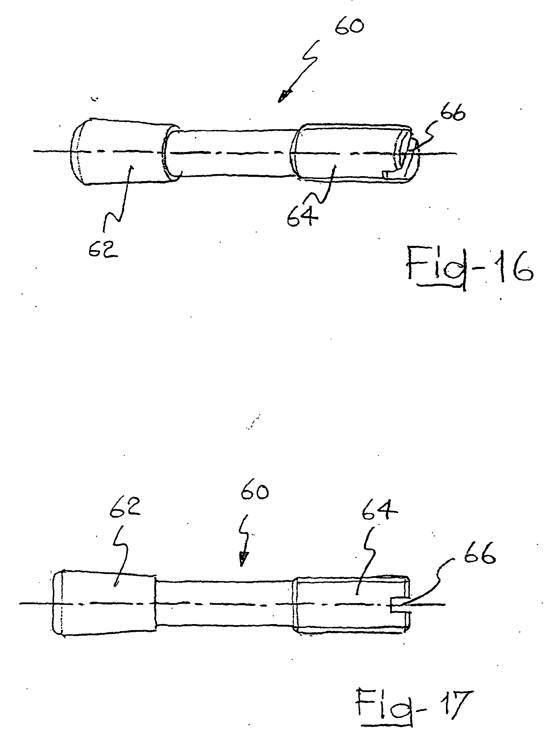

Figure 13 is a side-elevation view of the detail of figure 12.
Figure 14 is a cross section of the detail of figure 13, taken according to the traced C-C plane of figure 13.
Figure 15 is a front-elevation view of the detail of figure 12.
Figure 16 is an enlarged-scale perspective view of an element of the prosthesis of figure 1.
Figure 17 is a side-elevation view of the element of figure 16.

### Detailed description of a preferred embodiment

With reference to the drawings, an articular prosthesis, for metacarpus-phalangeal or interphalangeal use, according to the present invention and globally indicated with 10.

The prosthesis 10 comprises a first elongated portion or shank 12, to be associated for example to the proximal bone, which will be indicated hereafter also as proximal portion or shank 12, and a second elongated portion or shank 14, to be associated for example to the distal bone, which will be indicated hereafter also as distal portion or shank 14. In order to give an idea of the prosthesis 10 size, it can be pointed out that a typical length of these shanks 12 and 14 is 18 mm.

It is shown that it is alternatively possible to assemble the first portion 12 on the distal bone and the second portion 14 on the proximal bone, the prosthesis 10 of the invention always being of the same type as described hereafter.

The proximal shank 12 and the distal shank 14 are connected to each other by means of an articulated joint 16, equipped with two half parts 13 and 15 hinged to each other. More precisely, for example, this articulated joint 16 is formed by connecting a male end 13 of the proximal shank 12 to a female end 15 of the distal shank 14. The articulated joint 16 comprises also a pin 18, for example having a 3 mm diameter, hinging the two ends 13 and 15 to each other. At least a bush 20 (in the example the bushes 20 are two and they are located at two opposite sides of the male end 13) is interposed between facing walls of said two ends 13 and 15: this bush 20 is made of a low-friction and highly wearproof material, for example Peek Optima® or ultra-high-molecular-weight polyethylene UHMWPE or aluminium. The pin 18 has a reference head 68, with a recess 70 for centring the prosthesis 10, and stabilising a tool suitable to insert the pin 18. An opposite end to the reference head 68 is equipped with a convex surface, suitable to favour the abutment of a punch for extracting the pin 18.

The proximal portion 12, at an opposite end of the male end 13, can be inserted in an expansion insert 22, which is hollow and located in the metacarpal or phalangeal canal of the proximal bone. Similarly, the distal portion 14, at an opposite end of the female end 15, can be inserted in another expansion insert, of the same type as insert 22, which is hollow and located in the metacarpal or phalangeal canal of the distal bone.

The prosthesis 10 according to the invention can alternately provide the use of a single expansion insert 22, wherein the proximal 12 or distal 14 portion is inserted, the respectively opposite portion being traditionally fixed.

The insert 22 is preferably made of titanium and it is coated with hydroxyapatite in the bone contact areas, so that it can integrate with the bone.

More precisely, according to an aspect of the present invention, this insert 22, with a substantially cylindrical cavity 24 of axis X-X, has a slightly frustum conical external profile 26. The frustum of cone is flattened, near the wider base, in two axially opposite areas 28 and 29. In correspondence with the smaller base of the frustum of cone, the insert 22 is closed, while the wider base is open and it extends, with a conical divarication 30, in a substantially cylindrical collar 32. The collar 32 has a through opening 34, for example quadrilateral-shaped, preferably squared, greater in size than the cavity 24 of the insert 22 and in communication with said cavity 24.

The external profile 26 of the insert 22 has circumferential grooves 36. Moreover, on at least some height of the frustum conical profile 26, and particularly on the side towards the smaller base thereof, through notches 38 are realised. The notches 38 are realised according to notching planes being substantially parallel to the axis X-X of the cylindrical cavity 24 and tangent to the generating circumference of the cylinder of the cavity 24. Advantageously, these notches 38 can be four, equally spaced along the circumference.

As already mentioned, the proximal portion 12 has a male end 13: this end is formed by a substantially parallelepiped-shaped body 40, located in correspondence with an axis Y-Y of the proximal portion 12: the body 40 has a hole 42, having an axis K-K directed perpendicularly to the axis Y-Y and misaligned therefrom by a misalignment d, for example of 1 mm: the axis K-K is located in correspondence with an average value of the joint instantaneous rotation centre.

The proximal portion 12 extends, beyond the male end 13, with a substantially cylindrical beat collar 44. A preferably-squared-cross-section parallelepiped-shaped body 46 is provided at the bottom of said collar 44, suitable to be inserted in the through opening 34 of the insert 22.

For example beyond said parallelepiped-shaped body 46, a sleeve 48 is integral with the first shank 12: it is internally threaded, at least on the opposite side of the free end of the sleeve 48, and with a conical internal final part 50, countersunk towards the free end of the sleeve 48.

Through notches 52 parallel to the axis Y-Y, being for example four and equally spaced around the sleeve 48, are provided on at least a part of the sleeve 48, starting from the free end.

It is shown that the internal hole of the sleeve 48 orthogonally reaches the hole 42 of the body 40: moreover, the body 40, on a side being diametrally opposite to the area wherein the internal hole of the sleeve 48 reaches the hole 42, provides a through opening 42a connecting the hole 42 to the outside.

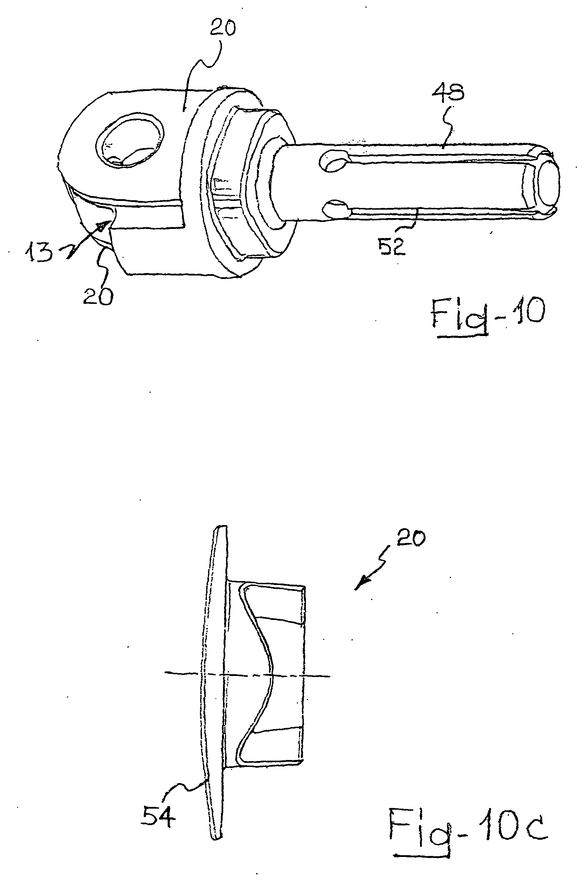

In the example, on each side of the hole 42, on the body 40, a bush 20 is provided, which is a holed plate: it is flat on the side abutting against the body 40 and it has a substantially cylindrical surface 54 on the opposite side, the axis of this cylinder being substantially perpendicular to the axis of the plate hole, coinciding in practise with the axis K-K of the hole 42. The plate hole is located in correspondence with the hole 42 of the proximal portion 12. More precisely, on the plate flat side, a substantially cylindrical collar 56, sized to be inserted in the hole 42, projects around this hole: the hole is internally frustum conical, countersunk towards the substantially cylindrical surface 54. Moreover, it is shown that a material release is preferably provided on a side of the substantially cylindrical collar 56, performed for example along an axis U-U misaligned by a misalignment u with respect to the axis K-K of the plate hole and passing through the plane of the frustum conical smaller base of the hole, as it is shown in figure 10e: as it can be noticed, once the bush 20 is assembled in the hole 42, the axis U-U is also parallel to the axis Y-Y of the proximal portion 12, a side surface 21 of the bush 20 abutting against a base of the cylinder of the beat collar 44.

As it has been mentioned, the distal portion 14 has a female end 15: this end has a pair of wings 41, located symmetrically with respect to an axis Z-Z of the distal portion 14: each wing 41 has a hole 43, directed according to an axis H-H perpendicularly to the axis Z-Z and misaligned therefrom by a misalignment h: the two holes 43 of the two wings 41 are opposite to each other and sized for the pin 18 and the misalignment h generally corresponds to the misalignment d of the hole 42. More precisely, the diameters of the two holes 43 are not identical: a hole 43 allows the pin 18 to be inserted with backlash, while the pin 18 engages in the other hole 43 with interference.

The distal portion 14 extends, beyond the female end 15, with a substantially cylindrical beat collar 45. A preferably-squared-cross-section parallelepiped-shaped body 47 is provided at the bottom of said collar 45, suitable to be inserted in the through opening 34 of the insert 22.

For example beyond said parallelepiped-shaped body 47, a sleeve 49 is integral with the second shank 14: it is internally threaded, at least on the opposite side of the free end of the sleeve 49, and with a conical internal final part 51, countersunk towards the free end of the sleeve 49.

Through notches 53 parallel to the axis Z-Z, being for example four and equally spaced around the sleeve 49, are provided on at least a part of the sleeve 49, starting from the free end.

It is shown that the internal hole of the sleeve 49 reaches an area of the portion 14 comprised between the two wings 41.

A profile 58 serving as a clamp is then provided between the two wings 41, on a side thereof, against an excessive rotation of the distal portion 14 with respect to the proximal portion 12. Preferably, a 90°-joint is ensured between the distal portion 14 and the proximal portion 12.

According to another aspect of the present invention, in each of the sleeves 48 and 49, to fasten the proximal 12 and distal 14 portions respectively in the corresponding expansion inserts 22, a rod-shaped element 60 is inserted. It has an externally threaded end 64 and, at the opposite end, it has a reverse-cone conical shank 62, i.e. with the cone being countersunk towards the free end. The rod-shaped element 60 has also, at the head of the end 64, a screwdriver notch 66 or another rotation-operating means of said rod-shaped element 60, being for example embedded-hexagon-shaped. The size of the thread of the end 64 corresponds to the internal threads of the sleeves 48 and 49: as it will be better seen hereafter, during the prosthesis 10 assembly, this notch 66, which can be accessed from the hole 42 of the body 40 and from the area of the portion 14 comprised between the two wings 41 respectively, serves to rotation-operate by means of a screwdriver the conical pin 60 which thus moves, in the respective sleeves 48 and 49, along the axes Y-Y and Z-Z (in the case of the embedded hexagon, a set screw wrench is obviously used). It must be noticed that the material release of the substantially cylindrical collar 56 serves to allow the notch 66 to be accessed from the hole 42 of the body 40 whereon the two bushes 20 have been located.

After all, each rod-shaped element 60, together with the sleeves 48 and 49 respectively, forms expansion means 59, said sleeve 48 or 49 being expanded by said rod-shaped element 60 and causing an expansion of the insert 22, when the sleeve 48 or 49 is inserted in the cavity 24 of the insert 22, said expansion being of the reversible type: these expansion means 59 are also removably connected to a half part 13 or 15 respectively of the articulated joint 16. More precisely, in the example, the sleeves 48 and 49 belong to the proximal 12 and distal 14 portions, wherein the male 13 and female 15 ends respectively, forming the articulated joint 16, are also formed.

The articular prosthesis operation, for metacarpus-phalangeal or interphalangeal use, according to the invention, is specified hereafter.

First of all, the two expansion inserts 22 are located in the metacarpal or phalangeal canal of the proximal and distal bones respectively.

An expansion element 60 is then inserted in each of the sleeves 48 and 49 respectively from the side of the threaded end 64, in order to screw the first threads of the threaded end 64 with the internal thread of the sleeves 48 and 49 respectively.

The frustum conical end 62 of each expansion element 60, so-preassembled to the proximal 12 and distal 14 portions respectively, is inserted in the cavity 24 of the respective inserts 22.

Operating now on each notch 66, by means of a convenient screwdriver, respectively from the hole 42 of the body 40, starting from the through opening 42a, and from the area of the portion 14 comprised between the two wings 41, the screwing of the threaded end 64 on the internal thread of the sleeves 48 and 49 respectively continues. The expansion element 60 thus moves axially along the axes Y-Y and Z-Z respectively, so that the conical shank 62 operates on the internal conical final parts 50 and 51 of the sleeves 48 and 49 respectively. By means of the notches 52 and 53, the sleeves 48 and 49 are elastically deformable and they compress the walls of the cavity 24. It is then very evident how the conical shanks 62 of the rod-shaped elements 60, operating the internal conical final parts 50 and 51 of the respective sleeves 48 and 49, make the sleeves 48 and 49 elastically deform in the radial direction, operating as reverse collets, or expansion collets, towards the inserts 22.

This compression of the sleeves 48 and 49 against the cavities 24 of the inserts 22, increasing until the beat collar 44 and 45 respectively abuts against the respective collars 32 of the inserts 22, causes an expansion of the external frustum conical profile 26 of the inserts 22. The expansion is increased by the presence of the notches 38. However, the particular arrangement of these notches 38 prevents large through ports from forming between the insert 22 outside and the insert 22 inside, preventing the bone tissue from increasing inside the inserts 22: in practise, opening an external part of the notch 38, an internal part of the notch 38 closes up on itself.

In practise, in a step immediately after the implant of the prosthesis 10, inserts 22 already have a good stability, due to the expansion of the sleeves 48 and 49: a fast rehabilitation is thus allowed. In time, the final osteointegration between the insert 22 and the bone occurs: this stable fastening is created in correspondence with circumferential grooves 36, which are deformed as a result of the expansion undergone by the external profile 26: after all, the bone tissue growth occurs in these grooves 36, fastening in a stable way the insert 22.

It is now evident that there is nothing but realising the articulated joint 16, by inserting the body 40 between the wings 41: the axis K-K of the hole 42 corresponds to the axis H-H of the holes 43, so that the pin 18 is arranged to connect in a stable way the male end 13 to the female end 15.

It is necessary to remember, in the connection step, the interposition of the two bushes 20, arranged with the cylindrical collar 56 in the hole 42 and with the side surface 21 abutting on the cylinder base of the beat collar 44 of the proximal portion 12.

In practise, it can be noted that, by means of the substantially cylindrical surfaces 54, the distal portion 14, with the axis Z-Z thereof rotating with respect to the axis Y-Y of the proximal portion 12, is capable of enduring small side rotations with respect to the natural rotation plane of the axis Z-Z around the axis Y-Y: for example side rotations of 5° on one side and 5° on the other side are endured.

By means of the described structure, torsions of the distal portion 14 on the proximal portion 12 are also advantageously prevented. In fact, it must be first considered that inserts 22 are flattened in the two opposite areas 28 and 29, similarly to the medullary anatomy; moreover, the parallelepiped-shaped bodies 46 and 47, inserted in the corresponding through openings 34 of the collars 32 of the inserts 22, serve as torsional constraint for the respective proximal 12 and distal 14 portions.

In case a disassembly of the prosthesis 10 is necessary because, for example, of damages by wear, it is advantageously possible to remove and replace the damaged components therefrom leaving the two inserts 22 in the proximal and distal bones respectively. In fact, once the pin 18 is removed, expansion elements 60 are gradually unscrewed: the sleeves 48 and 49 progressively loosen the compression on the walls of the cavity 24, until the proximal 12 and distal 14 portions are disengaged from the respective inserts 22.

Concerning the insert 22, it must be noticed that it is closed at the front just to prevent the bone from growing inside, making thus the sleeves 48 and 49 immovable.

Moreover, the conical divarication 30 of the inserts 22, located before the collar 32, prevents undesirable abrupt bone resorptions from forming.

The main advantage achieved by the articular prosthesis for metacarpus-phalangeal or interphalangeal use according to the present invention is that it allows an unusually fast prosthesis implant intervention.

A further advantage is to allow an easy prosthesis disassembly, with a subsequent functional replacement for example of worn components, without loosing the integration of the bone with the insert.

The just-described articular prosthesis for metacarpus-phalangeal or interphalangeal use can undergo some changes, all within the reach of the skilled in the art and falling within the scope of protection of the present invention, as defined by the following claims.

## Claims

1. Articular prosthesis (10) for metacarpus-phalangeal or interphalangeal use, comprising a first shank (12) to be associated to the proximal bone and a second shank (14) to be associated to the distal bone, said two shanks (12, 14) being connected to a half part (13, 15) of an articulated joint (16) respectively, said two half parts (13, 15) being hinged to each other, wherein at least one among said first shank (12) and said second shank (14) comprises an expansion insert (22) and expansion means (59) reversibly expanding said insert (22), said expansion means (59) being removably connected to a half part of said articulated joint (16), said expansion insert (22) has a substantially cylindrical cavity (24), **characterised in that** said expansion means (59) comprise a sleeve (48, 49) being integral with said shank (12, 14) and a rod-shaped element (60) which can be inserted in said sleeve (48, 49), said sleeve (43, 49) being internally threaded, at least on the opposite side of the free end of said sleeve (48, 49), and having a conical internal final part (50, 51) that is countereunk towards the free end of said sleeve (48, 49), said rod-shaped element (60) being equipped with an externally threaded end (64) suitable to engage in the inner thread of said sleeve (48, 49) and, at the opposite end, having a reverse-cone conical shank (62) suitable to engage in said conical final part (50, 51), said sleeve (48, 49) being inserted in said cavity (24) of said expansion insert (22).

2. Prosthesis (10) according to claim 1, **characterised in that** said articulated joint (16) is formed by connecting a male end (13) of said first shank (12) to a female end (15) of said second shank (14), said articulated joint (16) comprising also a pin (18) connecting the two male (13) and female (15) ends to each other.

3. Prosthesis (10) according to claim 2, **characterised in that** at least a bush (20) is interposed between facing walls of said two ends (13, 15).

4. Prosthesis (10) according to claim 3, **characterised in that** said bushes (20) are two and they are located at two opposite sides of the male end (13).

5. Prosthesis (10) according to claim 3, **characterised in that** said bushes (20) are made of a low-friction and highly wearproof material.

6. Prosthesis (10) according to claim 2, **characterised in that** said pin (18) has a reference head (68), with a recess (70) for centring said prosthesis (10), and stabilising a tool suitable to insert said pin (18), an opposite end to said reference head (68) being equipped with a convex surface, suitable to favour the abutment of a punch for extracting said pin (18).

7. Prosthesis (10) according to claim 2, **characterised in that** said first shank (12), at an opposite end of the male end (13), can be inserted in a first expansion insert (22), which is hollow and located in the metacarpal or phalangeal canal of the proximal bone, said second shank (14), at an opposite end of the female end (15), being suitable for insertion in a second expansion insert (22), which is hollow and located in the metacarpal or phalangeal canal of the distal bone.

8. Prosthesis (10) according to claim 1, **characterised in that** said expansion inserts (22) are made of titanium and they are coated with hydroxyapatite in the bone contact areas.

9. Prosthesis (10) according to claim 1, **characterised in that** said frustum of cone is flattened, near the wider base, in two axially opposite areas (28, 29), said insert (22) being closed in correspondence with the smaller base of the frustum of cone, the wider base of the frustum of cone being open and extending, with a conical divarication (30), in a substantially cylindrical collar (32).

10. Prosthesis (10) according to claim 9, **characterised in that** said collar (32) has a through opening (34) greater in size than the cavity (24) of said insert (22) and in communication with said cavity (24).

11. Prosthesis (10) according to claim 1, **characterised in that** on at least some height of the frustum conical profile (26) through notches (38) are realised.

12. Prosthesis (10) according to claim 11, **characterised in that** said notches (38) are realised according to notching planes being substantially parallel to the axis (X-X) of the cylindrical cavity (24) and tangent to the generating circumference of the cylinder of the cavity (24).

13. Prosthesis (10) according to claim 12, **characterised in that** said notches (38) are four, equally spaced along the circumference and they are located on the side towards the smaller base of the frustum of cone.

14. Prosthesis (10) according to claim 2, **characterised in that** said male end (13) is formed by a substantially parallelepiped-shaped body (40), located in correspondence with an axis (Y-Y) of the first shank (12), said body (40) having a hole (42), having an axis (Z-Z) directed perpendicularly to the axis (Y-Y) of the first shank (12) and misaligned therefrom by a misalignment (d), said axis (K-K) being located in correspondence with an average value of the joint instantaneous rotation centre.

15. Prosthesis (10) according to claim 14, **characterised in that** said first shank (12) extends, beyond the male end (13), with a substantially cylindrical beat collar (44), a parallelepiped-shaped body (46) being provided at the bottom of said collar (44), suitable to be inserted in the through opening (34) of the insert (22).

16. Prosthesis (10) according to claims 4 or 14, **characterised in that** said two bushes (20) are located, on each side of the hole (42), on the body 40, each of said bushes (20) being a holed plate, flat on the side abutting against the body (40) and having a substantially cylindrical surface (54) on the opposite side, the axis of said cylinder being substantially perpendicular to the axis of the plate hole, coinciding with the axis (K-K) of the hole (42).

17. Prosthesis (10) according to claim 16, **characterised in that** said plate hole is located in correspondence with the hole (42) of the first shank (12), a substantially cylindrical collar (56), sized to be inserted in the hole (42), projecting on the plate flat side around said hole, said hole being internally frustum conical, with the wider base towards the substantially cylindrical surface (54).

18. Prosthesis (10) according to claim 17, **characterised in that** a material release is provided on a side of the substantially cylindrical collar (56).

19. Prosthesis (10) according to claim 18, **characterised in that** said release is performed along an axis (U-U) misaligned by a misalignment (u) with respect to the axis (K-K) of the plate hole and passing through the plane of the frustum conical hole smaller base.

20. Prosthesis (10) according to claims 15 or 19, **characterised in that**, once the bush (20) is assembled in the hole (42), the release axis (U-U) is parallel to the axis (Y-Y) of the first shank (12), a side surface (21) of the bush (20) abutting against a base of the cylinder of the beat collar (44).

21. Prosthesis (10) according to claim 2, **characterised in that** said female end (15) has a pair of wings (41), located symmetrically with respect to an axis (Z-Z) of the second shank (14), each wing (41) having a hole (43), directed according to an axis (H-H) perpendicularly to the axis (Z-Z) of the second shank (14) and misaligned therefrom by a misalignment (h), said two holes (43) of the two wings (41) being opposite to each other and sized for the pin (18).

22. Prosthesis (10) according to claim 21, **characterised in that** the diameters of the two holes (43) are different, a hole (43) allowing the pin (18) to be inserted with backlash, the pin (18) engaging in the other hole (43) with interference.

23. Prosthesis (10) according to claim 21, **characterised in that** the second shank (14) extends, beyond the female end (15), with a substantially cylindrical beat collar (45), a parallelepiped-shaped body (47) being provided at the bottom of said collar (45), suitable to be inserted in the through opening (34) of the insert (22).

24. Prosthesis (10) according to claim 21, **characterised in that** a profile (58) serving as a clamp is provided between the two wings (41), on a side thereof, against an excessive rotation of the second shank (14) with respect to the first shank (12).

25. Prosthesis (10) according to claim 1, **characterised in that** said rod-shaped element (60) is threaded at the opposite end (64) to the conical shank (62) and ending, at the head, with a rotation-operating means (66) of said rod-shaped element (60)

26. Prosthesis (10) according to claim 25, **characterised in that** said rotation-operating means is a screwdriver notch (66) or an embedded hexagon.

27. Prosthesis (10) according to claims 14 or 26, **characterised in that** the size of said thread of the end (64) corresponds to the internal threads of the sleeves (48, 49), said rotation-operating means (66) being suitable for access from a through opening (42a) of the body (40) and from the area of the second shank (14) comprised between the two wings (41) respectively.

## Patentansprüche

1. Gelenkprothese (10) für metakarpal-phalangealen oder interphalangealen Gebrauch, mit einem ersten Schaft (12), der einem proximalen Knochen zuzuordnen ist, und einem zweiten Schaft (14), der einem distalen Knochen zuzuordnen ist, wobei beide Schäfte (12, 14) jeweils mit einem Halbteil (13, 15) einer Gelenkverbindung (16) verbunden sind, und die beiden Halbteile (13, 15) aneinander angelenkt sind, wobei der erste Schaft (12) und/oder der zweite Schaft (14) einen Expansionseinsatz (22) und eine Expansionseinrichtung (59) umfassen, die den Expansionseinsatz (22) reversibel ausdehnt, wobei die Expansionseinrichtung (59) abnehmbar mit einem Halbteil der Gelenkverbindung (16) verbunden ist, und der Expansionseinsatz (22) eine im Wesentlichen zylindrische Aushöhlung (24) hat, **dadurch gekennzeichnet, dass** die Expansionseinrichtung (59) eine mit dem Schaft (12, 14) einstückige Hülse (48, 49) und ein stabförmiges Element (60) aufweist, das in die Hülse (48, 49) eingesetzt werden kann, wobei die Hülse (48, 49) wenigstens an der dem freien Ende der Hülse (48, 49) entgegengesetzten Seite mit einem Innengewinde versehen ist, und ein konisches inneres Endteil (50, 51) hat, das zum freien Ende der Hülse (48, 49) hin mit einer Einsenkung versehen ist, wobei das stabförmige Element (60) mit einem mit Außengewinde versehenen Ende (64) ausgestattet ist, das dazu geeignet ist, in das Innengewinde der Hülse (48, 49) einzugreifen, und am entgegengesetzten Ende einen konischen Schaft (62) mit gegenteiliger Kegelform hat, der dazu geeignet ist, in das konische Endteil (50, 51) einzugreifen, wobei die Hülse (48, 49) in die Aushöhlung (24) des Expansionseinsatzes (22) eingesetzt ist.

2. Prothese (10) nach Anspruch 1, **dadurch gekennzeichnet, dass** die Gelenkverbindung (16) gebildet wird, indem ein Innenkopf (13) des ersten Schafts (12) mit einem Außenkopf (15) des zweiten Schafts (14) verbunden wird, wobei die Gelenkverbindung (16) auch einen Stift (18) umfasst, der den Innenkopf (13) mit dem Außenkopf (15) verbindet.

3. Prothese (10) nach Anspruch 2, **dadurch gekennzeichnet, dass** zwischen einander zugewandten Wänden der beiden Köpfe (13, 15) wenigstens eine Lagerbuchse (20) eingesetzt ist.

4. Prothese (10) nach Anspruch 3, **dadurch gekennzeichnet, dass** zwei Lagerbuchsen (20) vorgesehen sind, die an zwei entgegengesetzten Seiten des Innenkopfs (13) angeordnet sind.

5. Prothese (10) nach Anspruch 3, **dadurch gekennzeichnet, dass** die Lagerbuchsen (20) aus einem reibungsarmen und hoch verschleißfesten Material bestehen.

6. Prothese (10) nach Anspruch 2, **dadurch gekennzeichnet, dass** der Stift (18) einen Referenzkopf (68) mit einer Ausnehmung (70) zum Zentrieren der Prothese (10) hat, und zum Stabilisieren eines Werkzeugs, das zum Einsetzen des Stifts (18) geeignet ist, wobei ein entgegengesetztes Ende des Referenzkopfs (68) mit einer konvexen Fläche ausgestattet ist, die sich dazu eignet, die Abstützung eines Dorns zum Herausziehen des Stifts (18) zu begünstigen.

7. Prothese (10) nach Anspruch 2, **dadurch gekennzeichnet, dass** der erste Schaft (12) an einem entgegengesetzten Ende des Innenkopfs (13) in einen ersten Expansionseinsatz (22) eingesetzt werden kann, der hohl ist und im metakarpalen oder phalangealen Kanal des proximalen Knochens sitzt, wobei der zweite Schaft (14) an einem entgegengesetzten Ende des Außenkopfs (15) sich zum Einsetzen in einen zweiten Expansionseinsatz (22) eignet, der hohl ist und im metakarpalen oder phalangealen Kanal des distalen Knochens sitzt.

8. Prothese (10) nach Anspruch 1, **dadurch gekennzeichnet, dass** die Expansionseinsätze (22) aus Titan bestehen und an den Knochenkontaktflächen mit Hydroxyapatit beschichtet sind.

9. Prothese (10) nach Anspruch 1, **dadurch gekennzeichnet, dass** der Kegelstumpf nahe der breiteren Basis an zwei axial entgegengesetzten Bereichen (28, 29) abgeflacht ist, wobei der Einsatz (22) in Entsprechung mit der kleineren Basis des Kegelstumpfs geschlossen ist, wobei die breitere Basis des Kegelstumpfs offen ist und mit einer konischen Aufweitung (30) in einen im Wesentlichen zylindrischen Bund (32) übergeht.

10. Prothese (10) nach Anspruch 9, **dadurch gekennzeichnet, dass** der Bund (32) eine Durchgangsöffnung (34) hat, die größer als die Aushöhlung (24) des Einsatzes (22) ist und mit der Aushöhlung (24) in Verbindung steht.

11. Prothese (10) nach Anspruch 1, **dadurch gekennzeichnet, dass** zumindest über eine gewisse Höhe des kegelstumpfförmigen Profils (26) Durchgangsnuten (38) gebildet sind.

12. Prothese (10) nach Anspruch 11, **dadurch gekennzeichnet, dass** die Nuten (38) in Übereinstimmung mit Nutebenen gebildet sind, die im Wesentlichen parallel zur Achse (X-X) der zylindrischen Aushöhlung (24) liegen und tangential zur Mantelfläche des Zylinders der Aushöhlung (24).

13. Prothese (10) nach Anspruch 12, **dadurch gekennzeichnet, dass** vier Nuten (38) vorgesehen sind, die gleichmäßig entlang des Umfangs beabstandet sind und an der Seite sitzen, die zur kleineren Basis des Kegelstumpfs weist.

14. Prothese (10) nach Anspruch 2, **dadurch gekennzeichnet, dass** der Innenkopf (13) durch einen im Wesentlichen quaderförmigen Körper (40) gebildet ist, der in Entsprechung mit einer Achse (Y-Y) des ersten Schafts (12) angeordnet ist, wobei der Körper (40) eine Bohrung (42) mit einer Achse (Z-Z) hat, die senkrecht zur Achse (Y-Y) des ersten Schafts (12) ausgerichtet ist und um eine Abweichung (d) zu dieser versetzt angeordnet ist, wobei die Achse (K-K) entsprechend einem Mittelwert des Momentanrotationspols des Gelenks angesiedelt ist.

15. Prothese (10) nach Anspruch 14, **dadurch gekennzeichnet, dass** sich der erste Schaft (12) mit einem im Wesentlichen zylindrischen Schlagbund über den Innenkopf (13) hinaus erstreckt, wobei an der Basis des Bunds (44) ein quaderförmiger Körper (46) vorgesehen ist, der sich dazu eignet, in die Durchgangsöffnung (34) des Expansionseinsatzes (22) eingesetzt zu werden.

16. Prothese (10) nach Anspruch 4 oder 14, **dadurch gekennzeichnet, dass** am Körper (40) auf jeder Seite der Bohrung (42) jeweils eine Lagerbuchse (20) angeordnet ist, wobei jede der Lagerbuchsen (20) eine mit Bohrung versehene Platte ist, die auf der am Körper (40) anliegenden Seite flach ist und an der entgegengesetzten Seite eine im Wesentlichen zylindrische Fläche (54) hat, wobei die Achse des Zylinders, mit der Achse (K-K) der Bohrung (42) zusammenfallend, im Wesentlichen senkrecht zur Achse der Plattenbohrung liegt.

17. Prothese (10) nach Anspruch 16, **dadurch gekennzeichnet, dass** die Plattenbohrung in Übereinstimmung mit der Bohrung (42) des ersten Schafts (12) angeordnet ist, wobei ein im Wesentlichen zylindrischer Bund (56), der von der Größe her so bemessen ist, dass er in die Bohrung (42) eingesetzt werden kann, an der Flachseite der Platte um die Bohrung herum vorsteht, wobei die Bohrung innen kegelstumpfförmig ist und die breitere Basis an der im Wesentlichen zylindrischen Fläche (54) liegt.

18. Prothese (10) nach Anspruch 17, **dadurch gekennzeichnet, dass** an einer Seite des im Wesentlichen zylindrischen Bunds (56) ein Materialabtrag vorgesehen ist.

19. Prothese (10) nach Anspruch 18, **dadurch gekennzeichnet, dass** der Abtrag entlang einer Achse (U-U) erfolgt, die bezüglich der Achse (K-K) der Plattenbohrung um eine Abweichung (u) versetzt ist und durch die Ebene der kleineren Basis der kegelstumpfförmigen Bohrung verläuft.

20. Prothese (10) nach Anspruch 15 oder 19, **dadurch gekennzeichnet, dass**, wenn die Lagerbuchse (20) in die Bohrung (42) eingesetzt ist, die Löseachse (U-U) parallel zur Achse (Y-Y) des ersten Schafts (12) ist, wobei eine Seitenfläche (21) der Lagerbuchse (20) an einer Basis des Zylinders des Schlagbunds (44) anliegt.

21. Prothese (10) nach Anspruch 2, **dadurch gekennzeichnet, dass** der Außenkopf (15) ein Paar seitlicher Ansätze (41) hat, die symmetrisch in Bezug auf eine Achse (Z-Z) des zweiten Schafts (14) angeordnet sind, wobei jeder seitliche Ansatz (41) eine Bohrung (43) hat, die entsprechend einer zur Achse (Z-Z) des zweiten Schafts (14) verlaufenden Achse (H-H) ausgerichtet und davon um eine Abweichung (h) versetzt sind, wobei die beiden Bohrungen (43) der beiden seitlichen Ansätze (41) einander gegenüberliegen und in der Größe für den Stift (18) bemessen sind.

22. Prothese (10) nach Anspruch 21, **dadurch gekennzeichnet, dass** die Durchmesser der beiden Bohrungen (43) verschieden sind, wobei der Stift (18) in eine Bohrung (43) mit Spiel eingesetzt werden kann, und der Stift (18) an der anderen Bohrung (43) mit Übermaß angreift.

23. Prothese (10) nach Anspruch 21, **dadurch gekennzeichnet, dass** sich der zweite Schaft (14) mit einem im Wesentlichen zylindrischen Schlagbund (45) über den Außenkopf (15) hinaus erstreckt, wobei an der Basis des Bunds (45) ein quaderförmiger Körper (47) vorgesehen ist, der sich dazu eignet, in die Durchgangsöffnung (34) des Einsatzes (22) eingesetzt zu werden.

24. Prothese (10) nach Anspruch 21, **dadurch gekennzeichnet, dass** zwischen den zwei seitlichen Ansätzen (41) an einer Seite davon ein Profil (58) vorgesehen ist, das als Festlegung gegenüber einer übermäßigen Rotation des zweiten Schafts (14) zum ersten Schaft (12) dient.

25. Prothese (10) nach Anspruch 1, **dadurch gekennzeichnet, dass** das stabförmige Element (60) am dem konischen Schaft (62) entgegengesetzten Ende (64) mit einem Gewinde versehen ist und am Kopf mit einer Drehbetätigungseinrichtung (66) des stabförmiges Element (60) endet.

26. Prothese (10) nach Anspruch 25, **dadurch gekennzeichnet, dass** die Drehbetätigungseinrichtung ein Schraubendreherschlitz (66) oder ein Innensechskant ist.

27. Prothese (10) nach Anspruch 14 oder 26, **dadurch gekennzeichnet, dass** die Größe des Gewindes des Endes (64) dem Innengewinde der Hülsen (48, 49) entspricht, wobei die Drehbetätigungseinrichtung (66) dazu geeignet ist, Zugang durch eine Durchgangsöffnung (42a) des Körpers (40) und von dem Bereich des zweiten Schafts (14) her zu finden, der zwischen den beiden seitlichen Ansätzen (41) liegt.

## Revendications

1. Prothèse articulaire (10) pour une utilisation métacarpienne - phalangienne ou interphalangienne, comprenant une première tige (12) à associer à l'os proximal et une seconde tige (14) à associer à l'os distal, lesdites deux tiges (12, 14) étant respectivement reliées à une demi-pièce (13, 15) d'un joint articulé (16), lesdites deux demi-pièces (13, 15) étant articulées entre elles, dans laquelle au moins une parmi ladite première tige (12) et ladite seconde tige (14) comprend un insert installé par expansion (22) et un moyen d'expansion (59) expansant ledit insert (22) de manière réversible, ledit moyen d'expansion (59) étant relié de manière amovible à une demi-pièce dudit joint articulé (16), ledit insert installé par expansion (22) a une cavité sensiblement cylindrique (24), **caractérisée en ce que** ledit moyen d'expansion (59) comprend un manchon (48, 49) étant d'un seul tenant avec ladite tige (12, 14) et un élément en forme de tige (60) qui peut être inséré dans ledit manchon (48, 49), ledit manchon (48, 49) étant fileté de manière interne, au moins sur le côté opposé à l'extrémité libre dudit manchon (48, 49), et ayant une pièce finale interne conique (50, 51) qui est fraisée vers l'extrémité libre dudit manchon (48, 49), ledit élément en forme de tige (60) étant équipé d'une extrémité filetée de manière externe (64) adaptée pour venir en prise dans le filet interne dudit manchon (48, 49) et, à l'extrémité opposée, ayant une tige conique en cône inversé (62) adaptée pour venir en prise avec ladite pièce finale conique (50, 51), ledit manchon (48, 49) étant inséré dans ladite cavité (24) dudit insert installé par expansion (22).

2. Prothèse (10) selon la revendication 1, **caractérisée en ce que** ledit joint articulé (16) est formé en reliant l'extrémité mâle (13) de ladite première tige (12) à une extrémité femelle (15) de ladite seconde tige (14), ledit joint articulé (16) comprenant aussi une broche (18) reliant les deux extrémités mâle (13) et femelle (15) l'une à l'autre.

3. Prothèse (10) selon la revendication 2, **caractérisée en ce qu'**au moins une douille (20) est interposée entre les parois qui se font face desdites deux extrémités (13, 15).

4. Prothèse (10) selon la revendication 3, **caractérisée en ce que** lesdites douilles (20) sont au nombre de deux et se trouvent sur deux côtés opposés de l'extrémité mâle (13).

5. Prothèse (10) selon la revendication 3, **caractérisée en ce que** lesdites douilles (20) sont fabriquées dans un matériau à coefficient de frottement réduit et très résistant à l'usure.

6. Prothèse (10) selon la revendication 2, **caractérisée en ce que** ladite broche (18) a une tête de référence (68), avec une cavité (70) pour centrer ladite prothèse (10) et stabiliser un outil adapté pour insérer ladite broche (18), une extrémité opposée à ladite tête de référence (68) étant équipée d'une surface convexe, adaptée pour favoriser la butée d'un poinçon afin d'extraire ladite broche (18).

7. Prothèse (10) selon la revendication 2, **caractérisée en ce que** ladite première tige (12), à une extrémité opposée de l'extrémité mâle (13), peut être insérée dans un premier insert installé par expansion (22), qui est creux et se trouve dans le canal métacarpien ou phalangien de l'os proximal, ladite seconde tige (14), à une extrémité opposée de l'extrémité femelle (15), étant adaptée pour l'insertion d'un second insert installé par expansion (22), qui est creux et se situe dans le canal métacarpien ou phalangien de l'os distal.

8. Prothèse (10) selon la revendication 1, **caractérisée en ce que** lesdits inserts installés par expansion (22) sont en titane et recouverts d'hydroxyapatite dans les zones de contact osseux.

9. Prothèse (10) selon la revendication 1, **caractérisée en ce que** ledit tronc de cône est aplati, près de la base la plus large, dans deux zones axialement opposées (28, 29), ledit insert (22) étant fermé en correspondance avec la base la plus petite du tronc de cône, la base la plus large du tronc de cône étant ouverte et s'étendant, avec une divergence conique (30), dans un collier sensiblement cylindrique (32).

10. Prothèse (10) selon la revendication 9, **caractérisée en ce que** ledit collier (32) a une ouverture traversante (34) plus grande en taille que la cavité (24) dudit insert (22) et en communication avec ladite cavité (24).

11. Prothèse (10) selon la revendication 1, **caractérisée en ce que**, à au moins une certaine hauteur du profil tronconique (26), des encoches traversantes (38) sont réalisées.

12. Prothèse (10) selon la revendication 11, **caractérisée en ce que** lesdites encoches (38) sont réalisées selon des plans d'encoche sensiblement parallèles à l'axe (X-X) de la cavité cylindrique (24) et tangents à la circonférence de production du cylindre de la cavité (24).

13. Prothèse (10) selon la revendication 12, **caractérisée en ce que** lesdites encoches (38) sont au nombre de quatre, espacées de manière égale le long de la circonférence et elles se trouvent sur le côté vers la base la plus petite du tronc de cône.

14. Prothèse (10) selon la revendication 2, **caractérisée en ce que** ladite extrémité mâle (13) est formée par un corps sensiblement en forme de parallélépipède (40), situé en correspondance avec un axe (Y - Y) de la première tige (12), ledit corps (10) ayant un trou (42), ayant un axe (Z - Z) dirigé à la perpendiculaire de l'axe (Y - Y) de la première tige (12) et décalé de celui-ci par un décalage (d), ledit axe (K - K) se trouvant en correspondance avec une valeur moyenne du centre de rotation instantané du joint.

15. Prothèse (10) selon la revendication 14, **caractérisée en ce que** ladite première tige (12) s'étend, au-delà de l'extrémité mâle (13), avec un collier de battement sensiblement cylindrique (44), un corps en forme de parallélépipède (46) étant disposé à la base dudit collier (44), adapté pour être inséré dans l'ouverture traversante (34) de l'insert (22).

16. Prothèse (10) selon la revendication 4 ou la revendication 14, **caractérisée en ce que** lesdites deux douilles (20) se trouvent, de chaque côté du trou (42), sur le corps (40), chacune desdites douilles (20) étant une plaque trouée, plate du côté butant contre le corps (40) et ayant une surface sensiblement cylindrique (54) du côté opposé, l'axe dudit cylindre étant sensiblement perpendiculaire à l'axe du trou de plaque, coïncidant avec l'axe (K - K) du trou (42).

17. Prothèse (10) selon la revendication 16, **caractérisée en ce que** ledit trou de plaque se situe en correspondance avec le trou (42) de la première tige (12), un collier sensiblement cylindrique (56), calibré pour être inséré dans le trou (42), faisant saillie du côté plat de la plaque autour dudit trou, ledit trou étant tronconique de manière interne, la base la plus large étant vers la surface sensiblement cylindrique (54).

18. Prothèse (10) selon la revendication 17, **caractérisée en ce qu'**une détente de matériau est assurée d'un côté du collier sensiblement cylindrique (56).

19. Prothèse (10) selon la revendication 18, **caractérisée en ce que** ladite détente est réalisée le long d'un axe (U - U) décalé selon un décalage (u) par rapport à l'axe (K - K) du trou de plaque et passant à travers le plan de la base la plus petite du trou tronconique.

20. Prothèse (10) selon la revendication 15 ou la revendication 19, **caractérisée en ce que**, une fois que la douille (20) est assemblée dans le trou (42), l'axe de détente (U - U) est parallèle à l'axe (Y - Y) de la première tige (12), une surface latérale (21) de la douille (20) butant contre une base du cylindre du collier de battement (44).

21. Prothèse (10) selon la revendication 2, **caractérisée en ce que** ladite extrémité femelle (15) a une paire d'ailettes (41), disposées symétriquement par rapport à un axe (Z - Z) de la seconde tige (14), chaque ailette (41) ayant un trou (43), dirigé selon un axe (H - H) de manière perpendiculaire à l'axe (Z - Z) de la seconde tige (14) et décalé de celui-ci par un décalage (h), lesdits deux trous (43) des deux ailettes (41) étant opposés l'un à l'autre et calibrés pour la broche (18).

22. Prothèse (10) selon la revendication 21, **caractérisée en ce que** le diamètre des deux trous (43) est différent, un trou (43) permettant à la broche (18) d'être insérée avec un jeu mécanique, la broche (18) venant en prise dans l'autre trou (43) avec contact.

23. Prothèse (10) selon la revendication 21, **caractérisée en ce que** la seconde tige (14) s'étend, au-delà de l'extrémité femelle (15), avec un collier de battement sensiblement cylindrique (45), un corps en forme de parallélépipède (47) étant disposé à la base dudit collier (45), adapté pour être inséré dans l'ouverture traversante (34) de l'insert (22).

24. Prothèse (10) selon la revendication 21, **caractérisée en ce qu'**un profil (58) servant de fixation est disposé entre les deux ailettes (41), sur un côté de celles-ci, contre une rotation excessive de la seconde tige (14) par rapport à la première tige (12).

25. Prothèse (10) selon la revendication 1, **caractérisée en ce que** ledit élément en forme de tige (60) est fileté à l'extrémité opposée (64) sur la tige conique (62) et s'achève, au niveau de la tête, par un moyen opérant une rotation (66) dudit élément en forme de tige (60).

26. Prothèse (10) selon la revendication 25, **caractérisée en ce que** ledit moyen opérant une rotation est une encoche de tournevis (66) ou un hexagone encastré.

27. Prothèse (10) selon la revendication 14 ou la revendication 26, **caractérisée en ce que** la taille dudit filet de l'extrémité (64) correspond aux filets internes des manchons (48, 49), ledit moyen opérant une rotation (66) étant adapté pour être accessible depuis une ouverture traversante (42a) du corps (40) et depuis la zone de la seconde tige (14) comprise respectivement entre les deux ailettes (41).
